Europäisches Patentamt

European Patent Office

Office européen des brevets

(11) Numéro de publication: **0 399 158 B1**

(12) **FASCICULE DE BREVET EUROPEEN**

(45) Date de publication de fascicule du brevet: **22.02.95** (51) Int. Cl.6: **C12N 5/00**, A01C 1/06

(21) Numéro de dépôt: **90104878.5**

(22) Date de dépôt: **15.03.90**

(54) **Procédé de conservation d'embryons végétaux.**

(30) Priorité: **23.05.89 FR 8906738**

(43) Date de publication de la demande:
**28.11.90 Bulletin 90/48**

(45) Mention de la délivrance du brevet:
**22.02.95 Bulletin 95/08**

(84) Etats contractants désignés:
**AT BE CH DE DK ES FR GB GR IT LI LU NL SE**

(56) Documents cités:
**EP-A- 0 187 341**
**DD-A- 258 624**
**DE-A- 3 033 562**

**C.R. ACAD. SCI. PARIS vol. 306, Série III,
1988, pages 515-520, Paris, FR; F. ENGEL-
MANN et al.: "Effets du milieu de culture sur
la production d'embroides destinés à la
cryoconservation chez le palmier à huile
(Elaeis guineesis Jacq.)"**

**PLANT CELL REPORTS vol. 7, 1988, pages
407-409, Berlin, DE; D.G. CHARNE et al.: "Embryogenesis following cryopreservation in
isolated microspores of repeseed (Brassica
napus L.)"**

(73) Titulaire: **SOCIETE DES PRODUITS NESTLE
S.A.**
**Case postale 353**
**CH-1800 Vevey (CH)**

(72) Inventeur: **Florin, Bruno**
**9bis rue de la Victoire**
**F-37000 Tours (FR)**
Inventeur: **Lecouteux, Claude**
**49, rue Blanqui, Apt. 286**
**F-37700 St-Pierre Des Corps (FR)**
Inventeur: **Petiard, Vincent**
**28 Allée Côte Fleurie**
**F-37100 Tours (FR)**

CHEMICAL ABSTRACTS vol. 110, no. 19, 8 mai 1989, page 436, abrégé no. 169725r, Columbus, Ohio, US; I. DELVALLEE et al.: "Cryopreservation of immature maize embryos after freeze-hardening in the ear and in vitro"

PASCAL DATABASE abrégé no. 89-X-0111380; M. GALERNE et al.: "Survie de cals embryogènes d'épicea après congélation à196 C" & Ann. Rech. Syvic., 1988

CAB DATABASE abrégé no. 891605582; C. NOVAILLE et al.: "Artificial reeds: Dreams and reality" & Biofutur, no. 67, 1988

**Description**

La présente invention a trait à un procédé de conservation d'embryons végétaux somatiques ou zygotiques.

De nombreuses espèces peuvent être cryoconservées sous forme de suspensions cellulaires, de cals ou bien de méristèmes.

La conservation à basse température des embryons se justifie dans de nombreux cas, par exemple pour réguler la production de plantules lorsqu'elle est saisonnière ou pour assurer le maintien d'une lignée clonale.

La cryoconservation des embryons offre la possibilité de bloquer temporairement le développement des embryons, le temps nécessaire à leur transport jusqu'au semis ou à leur stockage, ainsi que la possibilité de création de banques de génotypes afin d'éviter une dépréciation progressive du patrimoine génétique.

Les embryons somatiques présentent certains avantages pour la multiplication des plantes. Ils proviennent en principe d'une seule cellule et donnent des plantes génétiquement uniformes. Les embryons somatiques présentent dès le début de leur formation une structure bipolaire: ils possèdent les deux méristèmes de tige et de racine nécessaires pour produire une plante. L'embryogénèse somatique apparaît donc comme une alternative intéressante pour la propagation des plantes: elle pourrait permettre la multiplication rapide d'espèces ayant un coût de production élevé, ou celle d'individus particulièrement performants provenant de cultures in vitro, ou celle de plantes transformées difficilement manipulables par voie sexuée, par exemple.

Les problèmes posés par la conservation des embryons sont complexes. Il s'agit en particulier de conserver vivants les deux pôles méristématiques susceptibles d'assurer la reprise de l'élongation et de l'organogénèse de la racine et de la tige.

Il existe différents procédés connus de cryoconservation d'embryons.

L'un de ces procédés consiste en une préculture des embryons sur un milieu enrichi en sorbitol, en proline ou en mannitol, suivie d'une imprégnation à 0°C dans le milieu de culture additionné de diméthylsulfoxyde et/ou de glycérol, puis d'une congélation induite et lente jusqu'à -40°C et enfin d'une congélation rapide dans l'azote liquide. La reprise de l'embryogénèse après décongélation nécessite l'ajout d'acide 2,4 dichlorophénoxyacétique au milieu de culture.

Un autre procédé, applicable à des embryons somatiques cultivés sur un milieu gélosé, consiste en une préculture d'une semaine sur un milieu enrichi en saccharose, suivie d'une congélation directe dans l'azote liquide des embryons placés à sec dans un cryotube.

Encore un autre procédé connu consiste en une préculture des embryons somatiques sur un milieu auquel on ajoute graduellement au moins 2,5% de diméthylsulfoxyde, suivie d'une congélation lente jusqu'à -100°C puis d'une congélation rapide dans l'azote liquide. La reprise de l'embryogénèse est facilitée par une culture sur un milieu contenant 0,1 mg l$^{-1}$ d'acide 2,4 dichlorophénoxyacétique.

Le rôle des substances cryoprotectrices, telles que le diméthylsulfoxyde ou le glycérol, est de prévenir la formation de la glace lors de la congélation. Toutefois l'utilisation de telles substances peut présenter certains inconvénients. D'une part, elles ne sont généralement pas facilement sublimables, et ne peuvent donc laisser espérer un traitement ultérieur de lyophilisation, d'autre part elles peuvent s'avérer cytotoxiques.

Le rôle des auxines comme l'acide 2,4 dichlorophénoxyacétique est de conserver les cellules à l'état indifférencié en phase de callogénèse, et de favoriser la reprise de la prolifération cellulaire après décongélation des embryons. Or l'arrêt par congélation du développement des embryons induit souvent une reprise anormale de leur croissance. Les auxines peuvent favoriser la formation d'embryons adventifs par division cellulaire lors de la reprise de l'embryogénèse, et augmenter les risques d'embryogénèse secondaire.

Afin de pallier les inconvénients de l'art antérieur, la présente invention a pour but de proposer un procédé de conservation d'embryons végétaux somatiques ou zygotiques permettant d'assurer l'arrêt par congélation du développement des embryons, puis la reprise du développement sans apparition d'autres formes de morphogénèse telles que l'embryogénèse secondaire ou la callogénèse.

A cet effet, le procédé selon la présente invention est caractérisé par le fait que:

- les embryons sont prétraités sur un milieu de prétraitement contenant un agent de pression osmotique, puis
- les embryons prétraités sont refroidis et congelés à une température comprise entre -15°C et -40°C.

Les embryons congelés à -15/-40°C peuvent être conservés à cette température, ou plongés ultérieurement dans un fluide frigorifique et conservés à une température plus basse.

Un avantage de ce procédé est d'éviter l'emploi de substances cryoprotectrices toxiques et/ou non sublimables.

Un autre avantage est d'éviter l'emploi d'auxines dans les milieux de culture utilisés avant congélation et après décongélation lors de la reprise de croissance.

Un autre avantage est de permettre la conservation à une température faible (-15/-40°C) donc facilement accessible (dans un congélateur ménager par exemple) sans avoir à employer de matériel coûteux.

Un autre avantage est de proposer un procédé de conservation rapidement et facilement réalisable.

Un autre avantage est de permettre une éventuelle lyophilisation ultérieure des embryons.

Le présent procédé de conservation permet d'obtenir des embryons ayant une reprise de croissance normale, ne nécessitant aucun apport de substance de croissance, et ne présentant pas de prolifération adventive, d'où une qualité améliorée de la semence produite. En effet, l'embryogénèse secondaire est préjudiciable à une distribution industrielle du produit car on peut obtenir des semences multigermes indésirables.

Les embryons utilisés dans la présente invention peuvent être de toute espèce et d'origine diverse. Ces embryons sont, par exemple, des embryons de carotte.

Les embryons peuvent être des embryons somatiques ou des embryons zygotiques.

Les embryons somatiques peuvent être obtenus par l'intermédiaire de suspensions cellulaires indifférenciées. Dans ce cas, des semences de parent d'hybride par exemple peuvent être mises à germer aseptiquement. Les hypocotyles peuvent être coupés puis placés sur un milieu de culture contenant des auxines. Les cals obtenus peuvent être ensuite dissociés dans un milieu de culture liquide. On obtient alors une suspension cellulaire indifférenciée dont les cellules, après plusieurs repiquages, peuvent être transférées sur un milieu de culture. Après une dizaine de jours, la suspension cellulaire peut être filtrée afin de ne retenir que les agrégats cellulaires de dimension désirée. Ces agrégats peuvent être cultivés sur un milieu de culture exempt d'auxine pendant quelques jours, afin d'induire la formation d'embryons.

Les embryons zygotiques peuvent être obtenus par prélèvement par dissection sur la graine au stade mature ou légèrement immature.

Les embryons somatiques et zygotiques obtenus peuvent être triés en fonction de leur stade de développement. On choisit de préférence les embryons aux premiers stades de leur développement, lorsqu'ils présentent une taille comprise entre environ 150 et 600 $\mu$m, car ils sont alors plus résistants à la congélation. Ces dimensions correspondent aux stades de développement de coeur (150-300 $\mu$m) ou de torpille (300 -600 $\mu$m) des embryons somatiques.

Les embryons obtenus sont ensuite prétraités sur un milieu de culture, dit milieu de prétraitement. Ce milieu peut être un milieu habituel dans le domaine de la culture d'embryons, tel qu'un milieu de Murashige et Skoog, par exemple, auquel on ajoute un agent de pression osmotique et auquel on peut également ajouter certaines substances organiques, telles que la vitamine B1, l'acide nicotinique ou l'adénine.

Il est nécessaire, lors d'un processus de cryoconservation, de prévenir la formation de glace intracellulaire qui, même si elle n'endommage pas directement les embryons pendant la congélation, peut induire une recristallisation destructrice pendant la décongélation. Dans le présent procédé, afin de réduire les éventuels dommages subis par les embryons, on les déshydrate partiellement grâce à un prétraitement sur un milieu contenant un agent de pression osmotique.

Cet agent peut être choisi par l'homme du métier parmi les substances susceptibles de pénétrer les tissus et d'assurer une bonne pression osmotique afin d'obtenir une déshydratation correcte de la cellule, sans en influencer la perméabilité membranaire. Cet agent ne doit pas, de plus, présenter de risque de toxicité vis-à-vis des embryons. Cet agent peut être un sucre, tel que le saccharose ou le tréalose, ou toute autre substance connue pouvant remplir les mêmes fonctions.

La concentration en agent de pression osmotique du milieu de prétraitement ne doit par être trop faible afin d'assurer une déshydratation correcte de la cellule.

Elle ne doit pas être non plus trop forte afin de ne pas détériorer les embryons ou empêcher leur reprise de croissance après la décongélation.

Dans une forme de réalisation préférée du présent procédé de conservation, le milieu de prétraitement contient du saccharose à une concentration comprise entre 75 et 190 gl$^{-1}$, et de préférence entre 100 et 150 gl$^{-1}$.

On a constaté que ce seul prétraitement suivi d'une étape de congélation aux températures indiquées, permet d'atteindre, de manière simple, efficace et sûre, le but recherché.

Le prétraitement peut durer de 30 secondes à 36 heures. Ce prétraitement ne doit pas être trop court afin de permettre à l'agent de pression osmotique de déshydrater partiellement les cellules et d'en accroître la résistance à la congélation.

Le prétraitement ne doit pas non plus durer trop longtemps. On peut observer une diminution de la résistance des embryons à la congélation, illustrée par une diminution du nombre d'embryons encore vivants après décongélation, lorsque le prétraitement dépasse 36 heures. Il est possible que lors d'un prétraitement prolongé, l'embryon subisse des modifications morphologiques qui pourraient amoindrir sa résistance à la congélation, ceci étant dû au fait que l'embryon continue de croître et atteint alors un stade moins compatible avec un traitement de congélation.

D'autre part, la diminution de l'action protectrice de l'agent de pression osmotique lors de longues durées de prétraitement pourrait aussi s'expliquer par une accoutumance de l'embryon au milieu de prétraitement, ce qui entraînerait une diminution de l'effet de la pression osmotique.

Le prétraitement peut s'effectuer de préférence à température ambiante, c'est-à-dire à environ 18-24°C, et sous un éclairement moyen de environ 150-250 lux, par exemple.

Les embryons prétraités sont refroidis puis congelés à une température comprise entre -15°C et -40°C. Ils sont congelés de préférence à une température de environ -20°C.

Cette congélation peut s'effectuer en transférant les embryons avec le milieu de prétraitement dans des cryotubes et en plaçant ces cryotubes dans un congélateur ménager par exemple.

La vitesse de refroidissement imposée lors de la congélation influe sur la rapidité d'apparition de la glace et le degré de déshydratation des embryons au moment de cette prise en glace. On a constaté qu'il est préférable que la vitesse de refroidissement, pour la zone de température située entre -6°C et -40°C, soit modérée, de l'ordre de 0,1-1°C par minute, afin de favoriser la reprise de croissance ultérieure des embryons. Pour le passage de la température ambiante à une température de -6°C, la vitesse de refroidissement peut être plus rapide, par exemple comprise entre 1 et 5°C par minute.

Les embryons congelés à une température comprise entre -15 et -40°C peuvent être conservés tels quels pendant au moins 1 mois.

Pour des durées de conservation supérieures, il est préférable de conserver les embryons à une température plus basse, par exemple dans un fluide frigorifique.

En effet, il semble qu'une conservation prolongée à -15/-40°C soit incompatible avec la reprise de croissance, donc la viabilité des embryons après décongélation. Il semble que la température de -15/-40°C soit trop élevée pour assurer un arrêt complet du métabolisme de l'embryon.

Si l'on désire une conservation prolongée, la congélation à -15/-40°C est à considérer comme une première étape qui peut être suivie d'une congélation dans un fluide frigorifique à plus basse température.

Dans ce cas, les embryons sont de préférence maintenus à -15/-40°C pendant un certain temps, de l'ordre de 18 -24h, afin que la congélation soit complète.

Les embryons peuvent être ensuite immergés directement dans un fluide frigorifique, par exemple dans un bain de méthanol à -70°C ou un bain d'azote liquide à -196°C, ou refroidis à une température très basse, par tout autre moyen.

Les embryons congelés peuvent alors être conservés pendant de longues périodes, pouvant durer plusieurs années. Les embryons congelés peuvent aussi être ultérieurement enrobés dans des polymères artificiels ou naturels, par exemple de type alginate, afin d'obtenir des semences artificielles.

La décongélation des embryons peut s'effectuer, par exemple, par trempage des cryotubes dans un bain-marie à 40°C pendant 2 minutes. Les tubes peuvent être retirés du bain-marie avant fusion complète de la glace, afin d'éviter une élévation de température trop rapide.

Après complète décongélation, les embryons peuvent être débarrassés du milieu de prétraitement par simple lavage, puis les embryons lavés peuvent être placés sur un milieu de culture habituel dans le domaine de la culture d'embryons, tel qu'un milieu de Murashige et Skoog par exemple.

Ce milieu peut contenir un substrat carboné assimilable, tel que du saccharose à une concentration de 1 à 10 gl$^{-1}$ par exemple.

Ce milieu de culture est remarquable par le fait qu'il est exempt d'auxines.

Après cette remise en culture, les embryons reprennent un développement comparable à celui d'embryons non congelés.

La survie après congélation et remise en culture se traduit par une croissance bipolaire des embryons. Il y a bien maintien de l'intégrité de la structure de l'embryon au cours de son développement ultérieur sans aucune autre forme de morphogénèse telle que callogénèse ou embryogénèse adventive.

Le présent procédé de conservation d'embryons permet donc bien d'obtenir d'une part la survie des embryons dans leur intégrité morphologique, d'autre part un bon maintien de leur capacité à régénérer une plantule.

La présente invention est illustrée plus en détail dans les exemples ci-après.

Ces exemples sont précédés par un exemple de préparation traditionnelle d'embryons somatiques, par la description d'un test de viabilité et par le tableau 1 qui donne la composition d'un milieu de prétraitement et de culture préféré.

Exemple de préparation d'embryons somatiques

Une suspension cellulaire indifférenciée de cellules de carottes Daucus carota L. est repiquée tous les 12 jours, à raison de 1 gramme de biomasse pour 100 ml de milieu, sur un milieu de culture liquide de Murashige et Skoog de composition identique à celle donnée dans le tableau 1, auquel on a ajouté 20 g l$^{-1}$ de saccharose et 0,1 mg l$^{-1}$ d'acide 2,4 dichlorophénoxyacétique.

Toutes les manipulations sont effectuées sous hotte à flux laminaire, dans des conditions aseptiques.

La suspension est placée sur un agitateur animé d'un mouvement giratoire excentré de 100 tours par minute, et est cultivée à 24°C, sous un éclairement de 200 lux avec une photopériode de 16 heures.

Après 8 à 10 jours de culture, la suspension cellulaire est filtrée afin de ne retenir que les agrégats cellulaires ayant une taille comprise entre 50 et 180 $\mu$m. Ces agrégats de faible taille représentent un stade proembryonnaire des embryons qui poursuivront leur développement jusqu'aux stades coeur, torpille et plantule. Les agrégats sont lavés et placés sur un milieu de Murashige et Skoog ne contenant pas d'acide 2-4 dichlorophénoxyacétique, à raison de environ 1,5.10$^3$ agrégats par ml de milieu.

Après 10 jours de culture, des embryons se sont formés. On filtre la suspension de manière à ne retenir que les embryons ayant une taille comprise entre 150 et 600 $\mu$m.

Test de viabilité

Un test de viabilité, rapide et simple dans sa mise en oeuvre, a été établi afin d'évaluer le taux de viabilité des embryons après congélation.

Parmi les différents critères utilisables pour apprécier le taux de viabilité des embryons, on retient principalement:
- l'augmentation de la taille des embryons et
- l'apparition d'une coloration chlorophyllienne.

L'appréciation de ces critères peut être effectuée de différentes manières, par exemple par dénombrement visuel ou à l'aide de tests biochimiques (test de coloration par exemple).

Selon le principe de ce test, les embryons décongelés sont placés sur un milieu de culture liquide. On relève après 10 jours de culture, le nombre d'embryons dont la taille a augmenté et présentant des signes de coloration chlorophyllienne.

Le rapport de ce nombre au nombre total d'embryons présents permet de déterminer le taux de viabilité des embryons.

Les embryons peuvent ensuite être placés sur un milieu de culture solide de même composition que le milieu liquide précédent afin de poursuivre leur développement vers le stade plantule.

Après 10 jours de culture sur ce milieu solide, on détermine le taux de régénération comme étant le rapport entre le nombre d'embryons ayant évolués vers le stade plantule au nombre total d'embryons.

Tableau 1

| Composition du milieu de Murashige et Skoog (pH 5,8-6) | |
|---|---|
| Macroéléments | mg l$^{-1}$ |
| NH$_4$NO$_3$ | 1650 |
| CaCl$_2$,2H$_2$O | 440 |
| MgSO$_4$,7H$_2$O | 370 |
| KNO$_3$ | 1900 |
| KH$_2$PO$_4$ | 170 |
| Microéléments | |
| CoCl$_2$ | 0,025 |
| CuSO$_4$,5H$_2$O | 0,025 |
| FeSO$_4$,7H$_2$O | 27,8 |
| Na$_2$-EDTA | 37,3 |
| MnSO$_4$,4H$_2$O | 22,3 |
| KI | 0,83 |
| Na$_2$MoO$_4$ | 0,25 |
| ZnSO$_4$,7H$_2$O | 10,6 |
| H$_3$BO$_3$ | 6,2 |
| Autres éléments | |
| Acide nicotinique | 5 |
| Thiamine (vit.B$_1$) | 5 |
| Adénine | 2 |
| + agent de pression osmotique (milieu de prétraitement) ou substrats carbonés assimilables (milieu de culture) | |

Exemple 1

Des embryons somatiques de carotte, obtenus comme décrit ci-dessus, au stade torpille, d'une taille moyenne de 550 µm, sont placés dans des boîtes de Pétri, sur un milieu de prétraitement liquide de Murashige et Skoog contenant 135 gl$^{-1}$ de saccharose et exempt d'auxines, à raison d'environ 100 embryons pour 10 ml de milieu.

Ces embryons sont prétraités pendant 1 heure, à 20°C et sous un éclairement de 200 lux.

Ils sont ensuite transférés dans des cryotubes contenant 1,8 ml de milieu de prétraitement. Les cryotubes sont placés dans un congélateur ménager, où leur contenu est refroidi à une vitesse de l'ordre de 1°C/min jusqu'à -6°C, puis à une vitesse de l'ordre de 0,4°C/min de -6°C à -20°C.

Après 24 heures à -20°C, les cryotubes sont séparés en deux lots. Le premier lot est décongelé, en plongeant les cryotubes congelés dans un bain-marie à 40°C, pendant 2 minutes. Le second lot est plongé dans l'azote liquide à -196°C où il reste 1 heure, avant d'être décongelé de la même manière que le premier lot.

Après totale décongélation, les embryons sont lavés du milieu de prétraitement et placés sur un milieu de culture liquide de Murashige et Skoog contenant 5 gl$^{-1}$ de saccharose et exempt d'auxines, pendant 10 jours. Ils sont ensuite transférés sur un milieu de culture solide, de même composition.

Des embryons témoins, n'ayant subi ni prétraitement, ni congélation sont placés sur les mêmes milieux de culture.

Après 10 jours de culture sur milieu solide, on compare la capacité des embryons à régénérer des plantules de carotte.

Parmi les embryons témoins, 46% sont aptes à régénérer une plantule. Pour les embryons congelés à -20°C et à -196°C, les taux de régénération sont de 44% et 43%.

La capacité des embryons à régénérer des plantules n'est donc pas altérée par la congélation s'ils ont préalablement subi un prétraitement.

EP 0 399 158 B1

Exemple 2

On sélectionne deux populations d'embryons somatiques de carotte Daucus carota L.

Une population est constituée d'embryons au stade coeur de taille moyenne de 300 $\mu$m, l'autre population est constituée d'embryons au stade torpille de taille moyenne de 500 $\mu$m.

On prélève 5 échantillons (A,B,C,D et témoin) contenant environ 100 embryons chacun sur chaque population d'embryons.

Les échantillons $A_c$, $B_c$ et $C_c$ d'embryons au stade coeur, et les échantillons $A_t$, $B_t$ et $C_t$ d'embryons au stade torpille sont prétraités sur un milieu de Murashige et Skoog contenant 135 gl$^{-1}$ de saccharose, pendant 1 heure, à 20°C et sous un éclairement de 200 lux.

Les échantillons $A_c$, $B_c$, $A_t$ et $B_t$ sont ensuite refroidis à -20°C à une vitesse de l'ordre de 0,5°C/min. Ils sont maintenus à -20°C pendant 24 heures.

Les échantillons $A_c$ et $A_t$ sont décongelés, alors que les échantillons $B_c$ et $B_t$ sont plongés dans l'azote liquide à -196°C où ils restent pendant 1 heure avant d'être décongelés.

Pour comparaison, les échantillons $C_c$ et $C_t$ sont directement plongés dans l'azote liquide à -196°C après le prétraitement. Ils sont décongelés après 1 heure.

Pour comparaison, les échantillons $D_c$ et $D_t$ sont refroidis à -20°C à une vitesse de l'ordre de 0,5°C/min, sans avoir subi de prétraitement. Ils sont décongelés après 24 heures.

Les échantillons témoin coeur et torpille ne subissent aucun prétraitement et aucune congélation.

Les différents échantillons d'embryons sont alors remis en culture sur un milieu liquide de Murashige et Skoog contenant 5 gl$^{-1}$ de saccharose.

Après 10 jours de culture, on obtient les résultats suivants:

Taux de viabilité des embryons (%)

## Echantillon

| Stade | A | B | comparaison C | comparaison D | Témoin |
|---|---|---|---|---|---|
| Coeur | 88 | 71 | 0 | 0 | 90 |
| Torpille | 84 | 67 | 0 | 0 | 91 |

On remarque qu'en l'absence de prétraitement, les embryons congelés à -20°C (D) ne survivent pas, alors qu'une imprégnation d'une heure dans le milieu de prétraitement permet la survie de presque tous les embryons de stade coeur et torpille congelés à -20°C (A).

Les embryons ne survivent pas non plus à une immersion directe dans l'azote liquide, même après un prétraitement d'une heure (C).

Les embryons congelés à -196°C après prétraitement et congélation à -20°C (B), présentent un taux de viabilité très satisfaisant.

Il est donc nécessaire, si l'on souhaite conserver les embryons à -196°C, de passer par les phases de prétraitement et de congélation à -20°C.

Les embryons sont ensuite placés sur un milieu de culture solide, de même composition que le milieu liquide.

Après 10 jours de culture, la plupart des embryons des échantillons A et B, ainsi que les échantillons témoins, ont évolué en plantule présentant à la fois une pointe racinaire et un apex cotylédonnaire chlorophyllien bien différenciés.

Ces embryons ne présentent aucune trace de callogénèse ou d'embryogénèse secondaire.

8

Exemple 3

Des embryons somatiques de carotte, au stade coeur et au stade torpille sont prétraités sur un milieu liquide de Murashige et Skoog contenant 135 gl⁻¹ de saccharose, à 20°C et sous un éclairement de 200 lux, pendant une durée variable.

Ces embryons sont ensuite refroidis à -20°C à une vitesse de l'ordre de 0,5°C/min. Après 24 heures à -20°C une partie est plongée dans l'azote liquide et y est maintenue pendant 1 heure.

Après décongélation, les embryons sont placés sur un milieu de culture liquide de Murashige et Skoog contenant 5 gl⁻¹ de saccharose.

Des embryons témoins n'ayant subi ni prétraitement, ni congélation sont aussi cultivés. On relève, après 10 jours de culture, le taux de viabilité des embryons.

On obtient les résultats suivants:

Taux de viabilité des embryons au stade coeur (%)

| | durée du prétraitement | | | | |
| | | | | comparaison | |
| | 30 min | 1h | 24h | 48h | 72h |
| --- | --- | --- | --- | --- | --- |
| Embryons congelés à -20°C | 89 | 88 | 87 | 56 | 40 |
| Embryons congelés à -196°C | 73 | 72 | 68 | 42 | 32 |

Les embryons témoins ont un taux de viabilité de 90%.

Taux de viabilité des embryons au stade torpille (%)

| | durée du prétraitement | | | | |
| | | | | comparaison | |
| | 30 min | 1h | 24h | 48h | 72h |
| --- | --- | --- | --- | --- | --- |
| Embryons congelés à -20°C | 85 | 84 | 82 | 56 | 34 |
| Embryons congelés à -196°C | 78 | 77 | 78 | 46 | 36 |

Les embryons témoins ont un taux de viabilité de 91%.

On remarque que le taux de viabilité après 10 jours de culture est quasi identique pour une période de prétraitement de 30 min, de 1h ou de 24h, aussi bien pour les embryons au stade coeur que pour les embryons au stade torpille.

EP 0 399 158 B1

Par contre, au-delà de 24h, le taux de viabilité diminue progressivement en fonction de l'allongement de la durée du prétraitement. Ce taux n'est plus que d'environ 35% après 72h de prétraitement. On observe donc une diminution de la résistance des embryons à la congélation lors des prétraitements prolongés.

## Exemple 4

Des embryons somatiques de carotte au stade torpille sont prétraités sur un milieu liquide de Murashige et Skoog contenant 135 gl$^{-1}$ de saccharose, à 20°C et sous un éclairement de 200 lux pendant une durée variable.

Ces embryons sont refroidis et congelés à -20°C à une vitesse de l'ordre de 0,5°C/min.

Après 72 heures à -20°C, les embryons sont décongelés et placés sur un milieu de culture liquide de Murashige et Skoog contenant 5 gl$^{-1}$ de saccharose.

Des embryons témoins n'ayant subi ni prétraitement, ni congélation sont aussi cultivés.

On relève, après 10 jours de culture, le taux de viabilité des embryons.

On obtient les résultats suivants:

| Durée du prétraitement (minute) | 1 | 15 | 30 | 45 | 60 | Témoin |
|---|---|---|---|---|---|---|
| Taux de viabilité (%) | 86 | 85 | 73 | 68 | 83 | 80 |

On remarque que le taux de viabilité des embryons après congélation à -20°C est bon pour toutes ces durées du prétraitement. La tolérance à la congélation des embryons s'acquiert donc très rapidement.

## Exemple 5

Des embryons somatiques de carotte au stade torpille, de taille moyenne de 550 $\mu$m sont prétraités et congelés à -20°C, une partie étant ensuite plongée dans l'azote liquide, selon la méthode décrite dans l'exemple 1.

Les embryons sont conservés à ces températures pendant un temps variable. Ils sont ensuite décongelés et placés sur un milieu de culture liquide de Murashige et Skoog contenant 5 gl$^{-1}$ de saccharose. On détermine le taux de viabilité des embryons en fonction de leur durée de conservation à -20°C ou -196°C.

Les embryons témoins n'ayant subi ni prétraitement, ni congélation ont un taux de viabilité de 72%.

On obtient les résultats suivants:

Pour les embryons congelés à -20°C:

| Durée de conservation | 24h | 1 mois | 2 mois |
|---|---|---|---|
| Taux de viabilité (%) | 64 | 69 | 0 |

Pour les embryons congelés à -196°C:

| Durée de conservation | 1h | 1 mois | 2 mois |
|---|---|---|---|
| Taux de viabilité (%) | 66 | 67 | 67 |

Le taux de viabilité moyen après congélation à -20°C ou à -196°C est d'environ 67% pour des durées de conservation d'un jour ou moins.

Ce taux reste inchangé après un mois de conservation à -20°C ou à -196°C.

Après 2 mois de conservation, on n'observe plus aucune survie parmi les embryons conservés à -20°C, alors que le taux de viabilité est toujours le même pour les embryons conservés à -196°C.

## Exemple 6

Des embryons somatiques de carotte, au stade torpille, d'une taille moyenne de 500 $\mu$m, sont prétraités sous un éclairement de 200 lux, à 20°C et pendant 1 heure sur des milieux liquides de Murashige et Skoog ayant une concentration en saccharose variant de 35 gl$^{-1}$ à 205 gl$^{-1}$.

10

Les embryons sont ensuite refroidis et congelés à -20°C. Après 1 heure à -20°C, les embryons sont décongelés et placés sur un milieu de culture liquide de Murashige et Skoog contenant 5 gl$^{-1}$ de saccharose.

Des embryons témoins, n'ayant subi ni prétraitement, ni congélation sont aussi cultivés.

On détermine le taux de viabilité des embryons après 10 jours de culture, en fonction de la concentration en saccharose du milieu de prétraitement.

On obtient les résultats suivants:

| Saccharose (gl$^{-1}$) | 35 | 68 | 100 | 135 | 170 | 205 | témoin |
|---|---|---|---|---|---|---|---|
| Taux de viabilité (%) | 22 | 41 | 77 | 82 | 65 | 14 | 88 |

Le taux de viabilité des embryons prétraités sur un milieu contenant 135 gl$^{-1}$ de saccharose est comparable à celui des embryons témoins.

Ce taux est encore assez élevé pour une concentration en saccharose de 100 ou 170 gl$^{-1}$.

La viabilité des embryons est altérée lorsque la concentration en saccharose devient trop élevée (205 gl$^{-1}$) ou trop faible (35 gl$^{-1}$).

**Revendications**

1. Procédé de conservation d'embryons végétaux, caractérisé en ce que les embryons sont prétraités sur un milieu de prétraitement contenant un agent de pression osmotique, puis les embryons prétraités sont refroidis et congelés à une température comprise entre -15 et -40°C.

2. Procédé selon la revendication 1, caractérisé en ce que l'agent de pression osmotique est du saccharose.

3. Procédé selon la revendication 2, caractérisé en ce que le milieu de prétraitement contient le saccharose à une concentration de 75-190 gl$^{-1}$.

4. Procédé selon la revendication 1, caractérisé en ce que les embryons congelés à -15/-40°C sont plongés dans un fluide frigorifique.

5. Procédé selon la revendication 1, caractérisé en ce que le prétraitement a une durée comprise entre 30 secondes et 36 heures.

6. Procédé selon la revendication 1, caractérisé en ce que la vitesse de refroidissement entre -6°C et -40°C est comprise entre 0,1 et 1°C/min.

7. Procédé selon la revendication 1, caractérisé en ce que le milieu de prétraitement est un milieu de Murashige et Skoog auquel on ajoute un agent de pression osmotique.

8. Application du procédé selon la revendication 1 , pour la production de plantules, caractérisée en ce que les embryons congelés sont décongelés et lavés du milieu de prétraitement, et les embryons lavés sont cultivés sur un milieu de culture, pour être développés en plantules.

9. Application du procédé selon la revendication 1 , pour l'obtention de semences artificielles, caractérisée en ce que les embryons congelés sont enrobés dans des polymères artificiels ou naturels.

10. Semence artificielle obtenue selon la revendication 9.

**Claims**

1. A storage process for vegetable embryos, characterized in that the embryos are pretreated on a culture medium containing an osmotic pressure agent and, after pretreatment, are cooled and frozen to a temperature in the range from -15°C to -40°C.

2. A process as claimed in claim 1, characterized in that the osmotic pressure agent is sucrose.

11

3. A process as claimed in claim 2, characterized in that the pretreatment medium contains the sucrose in a concentration of 75 to 190 gl$^{-1}$.

4. A process as claimed in claim 1, characterized in that the embryos frozen to -15/-40 °C are immersed in a liquid refrigerant.

5. A process as claimed in claim 1, characterized in that the pretreatment is carried out for 30 seconds to 36 hours.

6. A process as claimed in claim 1, characterized in that the cooling rate between -6 °C and -40 °C is between 0.1 and 1 °C/minute.

7. A process as claimed in claim 1, characterized in that the pretreatment medium is a Murashige and Skoog medium to which an osmotic pressure agent is added.

8. The application of the process claimed in claim 1 to the production of plantlets, characterized in that the frozen embryos are defrosted and washed to remove the pretreatment medium and the washed embryos are cultured on a culture medium for development into plantlets.

9. The application of the process claimed in claim 1 to the production of artificial seeds, characterized in that the frozen embryos are encapsulated in artificial or natural polymers.

10. Artificial seeds obtained in accordance with claim 9.

**Patentansprüche**

1. Verfahren zur Konservierung von pflanzlichen Embryonen, dadurch gekennzeichnet, daß die Embryonen auf einem Vorbehandlungsmedium, das ein Mittel für den osmotischen Druck enthält, vorbehandelt werden und dann die vorbehandelten Embryonen auf eine Temperatur von -15 bis -40 °C gekühlt und eingefroren werden.

2. Verfahren nach Anspruch 1, dadurch gekennzeichnet, daß das Mittel für den osmotischen Druck Saccharose ist.

3. Verfahren nach Anspruch 2, dadurch gekennzeichnet, daß das Vorbehandlungsmedium Saccharose in einer Konzentration von 75-190 gl$^{-1}$ enthält.

4. Verfahren nach Anspruch 1, dadurch gekennzeichnet, daß die auf -15/-40 °C eingefrorenen Embryonen in ein Kältemittel eingetaucht werden.

5. Verfahren nach Anspruch 1, dadurch gekennzeichnet, daß die Vorbehandlung eine Dauer von 30 Sekunden bis 36 Stunden hat.

6. Verfahren nach Anspruch 1, dadurch gekennzeichnet, daß die Geschwindigkeit der Abkühlung zwischen -6 °C und -40 °C 0,1 bis 1 °C/min beträgt.

7. Verfahren nach Anspruch 1, dadurch gekennzeichnet, daß das Vorbehandlungsmedium ein Murashige-Skoog-Medium ist, dem ein Mittel für den osmotischen Druck beigegeben wird.

8. Anwendung des Verfahrens nach Anspruch 1 auf die Herstellung von Sprossen, dadurch gekennzeichnet, daß die eingefrorenen Embryonen aufgetaut und durch Waschen von dem Vorbehandlungsmedium befreit werden und die gewaschenen Embryonen auf einem Kulturmedium kultiviert werden, um zu Sprossen entwickelt zu werden.

9. Anwendung des Verfahrens nach Anspruch 1 auf die Herstellung von künstlichem Saatgut, dadurch gekennzeichnet, daß die eingefrorenen Embryonen in künstliche oder natürliche Polymere eingehüllt werden.

**10.** Gemäß Anspruch 9 erhaltenes künstliches Saatgut.